# EUROPEAN PATENT APPLICATION

(11) **EP 4 445 961 A1**
(43) Date of publication of application: **16.10.2024**
(21) Application number: 22904302.1
(22) Date of filing: 08.12.2022
(51) Int. Cl.: A61Q 1/00, A61Q 1/10, A61K 8/02, A61K 8/06, A61K 8/81

(54) **AQUEOUS LIQUID COSMETIC**

(30) Priority: 08.12.2021 JP 2021199195
(71) Applicant: MITSUBISHI PENCIL COMPANY, LIMITED, Tokyo 140-8537 (JP)
(72) Inventor: INOUE Kensuke, Fujioka-shi, Gunma 375-8501 (JP)
(74) Representative: Vossius & Partner Patentanwälte Rechtsanwälte mbB
(86) International application number: PCT/JP2022/045312
(87) International publication number: WO 2023/106375

(57) **Abstract**

Provided is an aqueous liquid cosmetic that contains a pigment such as carbon black as a colorant, is capable of achieving, both in a highly balanced manner, cleansing property and fixing property of drawn lines even if the cosmetic does not contain a surfactant, and is suitable for makeup cosmetics such as eye shadow, eyeliner, eyebrow pencil, and mascara.

The aqueous liquid cosmetic according to the present disclosure is contained in an application coating tool that feeds liquid from an application liquid retaining part to an application part, by means of capillary force, and is characterized by containing at least: a pigment containing carbon as the main component; a water-soluble acrylate copolymer selected from group A; core-shell emulsion particles containing an acrylate copolymer; and an aqueous solvent.

Group A: (styrene/acrylate)copolymers and acrylate copolymer ammonium

## Description

### Technical Field

The present specification relates to an aqueous liquid cosmetic that contains a pigment such as carbon black as a colorant, is capable of achieving cleansing property and fixing property of drawn lines both in a highly balanced manner, even if the cosmetic does not contain a surfactant, and is suitable for makeup cosmetics such as eye shadow, eyeliner, eyebrow pencil, and mascara.

### Background Art

In the related art, as a typical makeup cosmetic composition, Patent Document 1 discloses an emulsion-type composition containing water and a lower polyhydric alcohol in an aqueous phase, and further containing an inorganic pigment such as iron oxide, a core-shell-type emulsion of an acrylate polymer, a hydrating oil, and a wax. This makeup cosmetic can be washed away with warm water while exhibiting a certain water resistance.

However, the cosmetic described in Document 1 contains a hydrating oil as an essential component in exchange for the fact that it can be washed away with warm water. Therefore, even if it is dried after coating, moisture remains in the coating film, and even if it has water resistance, there is a possibility that the fixation will be dissolved by moisture such as sweat or tears.

As a cosmetic composition suitable for an eyeliner, Patent Document 2 discloses a low-viscosity composition stored in a so-called collector-type applicator and containing an anionic surfactant such as sodium diethylhexyl sulfosuccinate, an inorganic pigment containing carbon black, a dispersant selected from water-soluble resins such as acrylate polymers, and a film-forming polymer emulsion which may be selected from core-shell type alkyl acrylate copolymer emulsions.

However, the cosmetic described in Document 2 contains sodium diethylhexyl sulfosuccinate as an essential component. In this case, when the cosmetic is dried after application, there is a possibility that even if it is washed, sodium diethylhexyl sulfosuccinate is not dissolved, and the fixed cosmetic is released.

Meanwhile, in recent years, in consideration of skin, "surfactant-free" (a composition free from a surfactant) makeup cosmetics are also increasing in the market in response to consumer's needs to avoid synthetic surfactants as much as possible.

### Citation List

### Patent Document

Patent Document 1: JP 2010-265225 A (Claims, Examples, etc.)
Patent Document 2: JP 2020-70259 A (Claims, Examples, etc.)

### Summary of Invention

### Technical Problem

The present disclosure is intended to solve the problems and current situation with regard to the aqueous liquid cosmetics of, described above as the related art. An object of the present disclosure is to provide an aqueous liquid cosmetic which contains a pigment such as carbon black as a colorant, which can highly achieve fixation and cleansing properties of drawn lines even if it does not contain a surfactant, and which is suitable as a makeup cosmetic such as an eyeshadow, an eyeliner, an eyebrow cosmetic, or a mascara. Solution to Problem

In consideration of the typical problems of mentioned above and as a result of diligent studies, the present inventors have found that an aqueous liquid cosmetic that achieves the above object can be obtained in the form of an aqueous liquid cosmetic stored in an applicator of a type in which liquid is fed from an application liquid storage portion to an application portion using capillary force, and containing at least a pigment containing carbon as a main component, a specific water-soluble acrylate copolymer, core-shell-type emulsion particles containing acrylate copolymer, and an aqueous solvent, and have completed the present disclosure.

That is, the aqueous liquid cosmetic of the present disclosure is a cosmetic stored in an applicator of a type in which liquid is fed from an application liquid storage portion to an application portion using capillary force, the cosmetic containing at least a pigment containing carbon as a main component, a water-soluble acrylate copolymer selected from Group A below, core-shell-type emulsion particles containing acrylate copolymer, and an aqueous solvent.

Group A: (styrene/acrylate) copolymer and ammonium acrylate copolymer. The pigment containing carbon as a main component is preferably selected from carbon black and/or graphite.

The aqueous solvent is preferably selected from water, a lower alcohol having 6 or less carbons, or a polyhydric alcohol.

The content of water is preferably 50 mass% or more.

The application portion of the applicator is preferably composed of a brush.

Further, it is preferable to contain an inorganic pigment other than the iron oxide pigment.

As used herein, the term "aqueous" refers to a composition of a single system (not a W/O emulsion, an O/W emulsion, or the like) containing water and/or a water-soluble organic solvent as a main solvent. Advantageous Effects of Invention

According to the disclosure, there is provided an aqueous liquid cosmetic which contains a pigment such as carbon black as a colorant, which can highly achieve fixation and cleansing properties of drawn lines even if it does not contain a surfactant, and which is suitable as a makeup cosmetic such as an eyeshadow, an eyeliner, an eyebrow cosmetic, or a mascara.

The object and effects of the present disclosure can be recognized and obtained especially using the components and combinations indicated in the claims. Both general explanation described above and detailed explanation described below are exemplary and explanatory and do not limit the present disclosure described in claims.

### Brief Description of Drawings

FIG. 1 is a vertical cross-sectional view illustrating an example of a liquid cosmetic applicator that stores an aqueous liquid cosmetic of the disclosure and illustrating a state before use.
FIG. 2 is a vertical cross-sectional view illustrating the liquid cosmetic applicator of FIG. 1 at the start of use.
FIG. 3 is a partial vertical cross-sectional view illustrating another example of a liquid cosmetic applicator that stores the aqueous liquid cosmetic of the disclosure.
FIG. 4 is a partial cross-sectional view illustrating another example of a liquid cosmetic applicator that stores the aqueous liquid cosmetic of the disclosure.

### Description of Embodiments

Embodiments of the present disclosure will be described below in detail. However, it should be noted that the technical scope of the present disclosure is not limited to the embodiments detailed below and includes the invention described in claims and equivalents thereof. In addition, the present disclosure can be implemented based on the contents disclosed in the present specification and technical common knowledge (including design matters and obvious matters) in the art.

The aqueous liquid cosmetic of the present disclosure is a cosmetic stored in a type of applicator in which liquid is fed from an application liquid storage portion to an application portion using capillary force, and the cosmetic contains at least a pigment containing carbon as a main component, a water-soluble acrylate copolymer selected from Group A below, core-shell-type emulsion particles containing acrylate copolymer, and an aqueous solvent.

Group A: styrene acrylate copolymer, styrene acrylic acid copolymer, and methylstyrene-acrylic acid copolymer.

Examples of the pigment containing carbon as a main component (carbon-based black pigment) used in the present disclosure include at least one (alone or a mixture of two or more types, the same shall apply hereinafter) of carbon black, graphite, or spherical graphite. One selected from carbon black and/or graphite is preferred, and carbon black is particularly preferred.

The particle shape of the pigment containing carbon as a main component is not particularly limited and may be any shape such as a spherical, granular, rod-like, needle-like, plate-like, or amorphous shape. The average particle size of the pigment (particles) containing carbon as a main component that can be used before dispersion (at the time of blending) varies depending on the application of the cosmetic and the type of pigment. For example, carbon black having an average particle size of 2000 nm or less, preferably 100 to 1000 nm, can be used.

In the present disclosure (including Examples), the "average particle size" refers to a value measured and calculated by a dynamic light scattering method [particle size analyzer FPAR-1000, available from Otsuka Electronics Co., Ltd.].

The (total) content of the pigment containing carbon as a main component is preferably 1 to 20 mass%, and more preferably 4 to 10 mass%, relative to the entire amount of the cosmetic composition.

When the content of the pigment containing carbon as a main component is less than 1 mass%, the color development is insufficient and the concealing power is also insufficient. Meanwhile, when the content exceeds 20 mass%, the viscosity is increased and writing is hindered, which is not preferred.

Examples of the water-soluble acrylate copolymer used in the present disclosure include at least one selected from Group A:
(styrene/acrylate) copolymer and ammonium acrylate copolymer.

Examples of the acrylate copolymer includes a copolymer composed of two or more of alkyl acrylate (C1 to C4), alkyl methacrylate (C1 to C4), acrylic acid, and methacrylic acid. Examples of commercially available products of the polymer include SYNTRAN EX149PE (available from INTERPOLYMER).

Examples of the ammonium acrylate copolymer include an alkyl acrylate copolymer. Examples of commercially available products of this polymer include SYNTRAN 5402 (available from INTERPOLYMER).

The content of such a water-soluble acrylate copolymer is 0.1 to 5.0 mass%, preferably 0.2 to 3.0 mass%, and more preferably 0.5 to 2.0 mass%, relative to the entire amount of the liquid cosmetic.

When the content of the water-soluble acrylate copolymer is 0.1 mass% or more, dispersibility and dispersion stability of the pigment can be improved. Meanwhile, when the content thereof is 5.0 mass% or less, an increase in the viscosity of the liquid cosmetic can be prevented, and good application performance can be achieved.

In the present disclosure, the core-shell-type emulsion particles containing the acrylate copolymer may be a random copolymer, a graft copolymer, a block copolymer, or a core-shell-type copolymer. In particular, an acrylate resin emulsion or a urethane-acrylate composite resin emulsion is preferred, wherein the emulsion contains particles having a core-shell structure in which the core is an acrylate resin such as poly(meth)acrylate or a urethane resin such as polyurethane and the shell is an acrylate resin such as poly(meth)acrylate or a urethane resin such as polyurethane.

Specific examples thereof include EMUPOLY-CE-119N (available from Gifu Shellac Mfg. Co., Ltd.) which is an emulsion of core-shell-type copolymer of ethylhexyl acrylate/methacrylate copolymer [(acrylate/methylstyrene/styrene) copolymer ammonium].

From the viewpoint of film-forming properties and fixation of drawn lines, it is preferable to contain acrylate copolymer, and more preferably, it is desirable to use the core-shell-type polymer emulsion described above.

In these emulsion particles, a resin component is usually finely dispersed in an aqueous component at a concentration of 20 to 60 mass% as a solid content. The blended amount thereof is 1 to 40 mass%, preferably 3 to 20 mass%, and more preferably 5 to 15 mass% in terms of solid content concentration relative to the entire amount of the liquid cosmetic.

When the content of the emulsion particles is less than 1 mass%, the fixation and the water-resistant fixation are lowered. Meanwhile, when the content thereof is more than 40 mass%, the viscosity of the liquid cosmetic is increased, leading to difficulty in application of the cosmetic.

The aqueous solvent used in the present disclosure is used as a solvent of a liquid cosmetic. Examples thereof include at least one (alone or a mixture of two or more types, the same shall apply hereinafter) of water, a lower alcohol having 6 or less carbons, or a polyhydric alcohol. Specific examples of the lower alcohol having 6 or less carbons include at least one of methyl alcohol (methanol), ethyl alcohol (ethanol), n-propyl alcohol, isopropyl alcohol, n-butyl alcohol, sec-butyl alcohol, tert-butyl alcohol, isobutyl alcohol, pentyl alcohol, ethylene glycol, or propylene glycol.

Examples of the polyhydric alcohol include glycerin, erythritol, threitol, arabinitol, xylitol, ribitol, propylene glycol, 1,3-butylene glycol, 1,2-pentanediol, ethylene glycol, diethylene glycol, and pentaerythritol.

In particular, it is desired to use ethyl alcohol (ethanol), from the perspective of safety and handleability.

The total content of the aqueous solvent to be used is preferably 45 to 85 mass%, and more preferably 50 to 80 mass%, relative to the entire amount of the liquid cosmetic, in terms of safety and dispersion stability of the pigment containing carbon as a main component, and particularly in terms of stability at low temperatures.

When the content of the aqueous solvent is 45 mass% or more, smooth comfort writing and moisture retaining properties can be achieved, and an antiseptic effect can be slightly achieved. Meanwhile, when the content thereof is 85 mass% or less, the bleeding resistance of a drawn line is further improved.

Water serving as a solvent used in the present disclosure can be distilled water, ion-exchanged water, purified water, pure water, or ultrapure water. In terms of further improving stability over time and dispersibility of the carbon black, the ratio of water to the aqueous solvent (water + a solvent other than water) is preferably 50 to 100 mass% (0.5 to 1.0), and particularly preferably 80 to 95 mass%.

Further, the aqueous liquid cosmetic of the present disclosure may contain, in addition to the above-described components, an optional component used in ordinary liquid cosmetics. Specifically, the aqueous liquid cosmetic may contain a predetermined amount of a preservative, an antioxidant, a neutralizing agent, an ultraviolet absorber, a chelating agent, a humectant such as 1,3-butylene glycol, a beauty ingredient, a perfume, a viscosity/stickiness modifier, or another dispersant such as polyethylene glycol alkyl ether as long as the effects of the present disclosure are not impaired. Besides the inorganic pigment other than the pigment containing carbon as a main component, an additional colorant such as an organic pigment, a water-soluble dye, or a resin particle pigment may optionally be contained, as long as the effects of the present disclosure are not impaired.

Examples of the inorganic pigment that can be used besides the pigment containing carbon as a main component include iron oxide particles (such as black iron oxide particles and yellow iron oxide particles), chromium oxide, ultramarine blue, iron blue, zinc oxide, aluminum oxide, silicon dioxide, titanium oxide, magnesium oxide, chromium hydroxide, calcium carbonate, titanium yellow, iron oxide red, and pearl pigments such as mica, titanated mica, carmine coated titanated mica, carmine/iron blue coated titanated mica, black iron oxide coated titanated mica, black iron oxide/carmine coated titanated mica, black iron oxide/iron blue coated titanated mica, blue coated titanated mica, iron oxide red coated mica, iron oxide red coated titanated mica, iron oxide red/carmine coated titanated mica, iron oxide red/black iron oxide coated titanated mica, iron oxide red/iron blue coated titanated mica, iron oxide red/black iron oxide/iron blue coated titanated mica, metal oxide coated glass powder, titanium oxide coated glass powder, iron oxide coated glass powder, silver coated glass powder, gold coated glass powder, and aluminum powder.

Examples of the organic pigment include Blue No. 1 Al lake, Red No. 202, Red No. 226, Red No. 228, Blue No. 404, Red No. 220, Yellow No. 401, Yellow No. 205, Blue No. 201, Blue No. 204, Yellow No. 4 Al lake, Yellow No. 203 Al lake, and Red No. 104 Al lake.

These coloring materials (such as inorganic pigments and organic pigments) other than the pigment containing carbon as a main component may be used alone, or two or more thereof may be used in a combination.

The aqueous liquid cosmetic of the present disclosure preferably has a viscosity of 15 mPa·s or less, preferably 10 mPa·s or less, and more preferably 2 to 8 mPa·s.

When the viscosity exceeds 15 mPa·s, the flowability of the aqueous liquid cosmetic is significantly decreased, which is not preferred. Adjustment of the above viscosity range can be performed, for example, by suitably combining the respective components such as the pigment containing carbon as a main component to be used and its average particle size, the water-soluble acrylate copolymer, the emulsion particles containing acrylate copolymer, and the aqueous solvent, by combining the contents of the respective components in a suitable range, or by a suitable dispersion method.

In the present disclosure, regarding the viscosity measurement conditions (including Examples described below), specifically, the resultant cosmetic composition is subjected to measurement with an ELD-type viscometer available from Toki Sangyo Co., Ltd using a standard rotor: 50 rpm (shear rate: 192 [s⁻¹]) at a temperature of 25°C.

Furthermore, the aqueous liquid cosmetic of the present disclosure has a surface tension at a temperature of 25°C preferably in a range of 35 to 60 mN/m, more preferably in a range of 35 to 45 mN/m as measured by the Wilhelmy method (plate method), from the viewpoint of good spreadability during application. When the surface tension is 35 mN/m or more, smooth application can be performed without bleeding or unevenness in applied lines, and dripping (dropping) is reduced even in the case where the cosmetic has a low viscosity. Meanwhile, when the surface tension is 60 mN/m or less, followability of the application liquid is improved and smooth application can be performed.

In the present disclosure (including Examples described below), the measured surface tension refers to a value obtained at a temperature of 25°C using a CBVP-Z type surface tensiometer (plate method) available from Kyowa Interface Science Co., Ltd.

The surface tension can be adjusted to fall within the above range by suitably combining the amounts of the respective components such as a pigment containing carbon as a main component, such as carbon black, a water-soluble acrylate copolymer, and emulsion particles containing acrylate copolymer.

When the aqueous liquid cosmetic of the present disclosure is prepared by using the above-mentioned pigment containing carbon as a main component, such as carbon black, water-soluble acrylate copolymer, emulsion particles containing acrylate copolymer, aqueous solvent, and additional component under suitable dispersion conditions using, for example, a dispersing machine such as a homomixer, a sand mill, an ultrasonic homogenizer, or a high-pressure homogenizer, the average particle size of carbon black in the cosmetic (after dispersion) can be adjusted to 200 nm or less.

Preferably, when a (multiple type) ultrasonic homogenizer or a high-pressure homogenizer is used as the dispersion machine, the average particle size of the pigment containing carbon as a main component in the cosmetic (after dispersion) can be adjusted to 200 nm or less by setting the dispersion conditions; for example, the frequency is set to 20 to 30 KHz in the case of an ultrasonic homogenizer, or the pressure is set to 150 to 245 MPa in the case of a high-pressure homogenizer.

The resultant aqueous liquid cosmetic can be stored in a type of applicator in which liquid is fed from an application liquid storage portion to an application portion using capillary force. For example, the aqueous liquid cosmetic can be stored (charged) in a pen-type liquid cosmetic applicator (container) having a brush or a pen core as an application portion for use.

The liquid cosmetic applicator that can be used is not particularly limited as long as it is a liquid cosmetic applicator equipped with a brush or a pen core for, for example, an eyeliner or an eyebrow pen. Preferred examples of the liquid cosmetic applicator include an applicator having, as application means, a brush (brush pen) or a pen core for an eyeliner or an eyebrow pen, or an application body formed of rubber, an elastomer, or a closed-cell foam having restorability, and having a container for charging the liquid cosmetic.

The aqueous liquid cosmetic material of the present disclosure is stored in a liquid cosmetic applicator having an application portion provided with a brush or a pen core for use.

In the present disclosure, a suitable liquid cosmetic applicator is not particularly limited as long as it is an eyeliner, an eyeshadow pen, an eyebrow pen suitable for point makeup around eyes, or a liquid cosmetic applicator having a structure capable of suitably applying a liquid cosmetic composition (application liquid) to the skin. Preferred examples of the liquid cosmetic applicator include an liquid cosmetic applicator including an inner cotton-type container configured to hold a liquid cosmetic in an inner cotton or a collector-type container configured to hold a liquid cosmetic in a sheet body, and a liquid cosmetic applicator having an application liquid storage portion that directly stores a liquid cosmetic composition and a knock-type or rotary (pivotal) discharging mechanism of the application liquid, which are excellent in usability, convenience, and application properties.

In the present disclosure, a preferred liquid cosmetic applicator is a liquid cosmetic applicator including at least an application portion and an application liquid storage portion, wherein the application portion is formed of a brush, the brush includes fibers having a cross section with a shape other than a circular shape, and the liquid cosmetic composition of the present disclosure is stored in the application liquid storage portion.

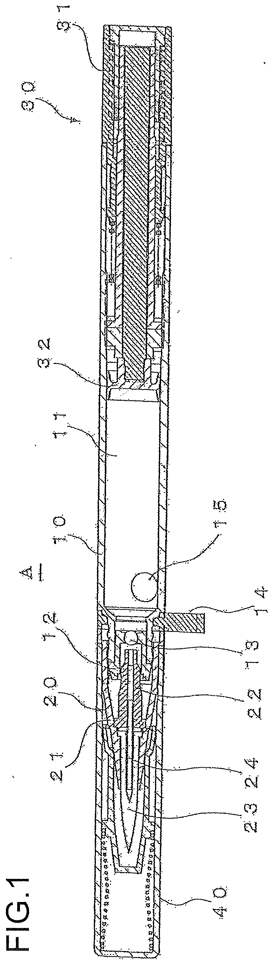

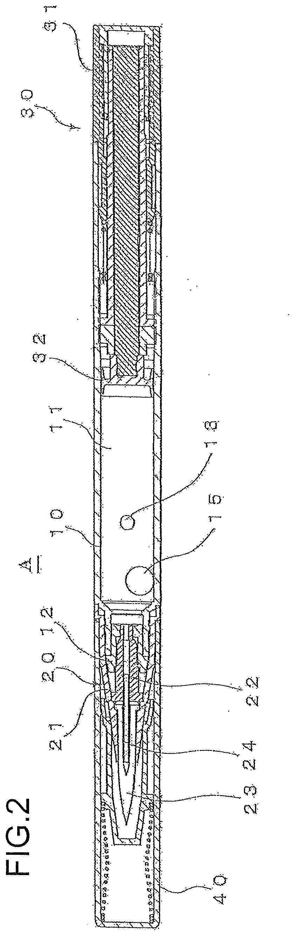

Examples of the liquid cosmetic applicator of this embodiment include a liquid cosmetic applicator illustrated in FIGS. 1 and 2.

As illustrated in FIG. 1, the liquid cosmetic applicator A according to the embodiment of the present disclosure includes a reservoir (storage portion)011 of an application liquid that is the liquid cosmetic composition of the present disclosure, which has the above structure and is disposed inside of the front of the barrel body 10 serving as the applicator body; a plug body 12 mounted on the front end portion of the barrel body 10 and is configured to seal the reservoir (storage portion) 11 before start of use; and a sealing ball 13. The sealing ball 13 is mounted to the plug body 12, and maintains the sealing condition of the reservoir (storage portion) 11. When the sealing ball 13 is removed from the plug body 12, the sealing condition of the reservoir (storage portion) 11 is released, to thereby allow the application liquid to be discharged from the reservoir (storage portion) 11.

The liquid cosmetic applicator A also includes a stopper 14 removably mounted on the outer peripheral surface of the front end portion of the barrel body 10; and a front barrel 20 that moves in the axial direction by the length of the stopper 14 and is directly joined to the barrel body 10 when the stopper 14 is removed at the start of use.

The front barrel 20 includes a front barrel body 21; a pipe joint 22 that is mounted on the front barrel body 21 and is capable of supplying the application liquid from the reservoir 11 to the brush (application portion) 23; and an application liquid conducting tube 24 connecting the pipe joint 22 and the brush (application portion) 23.

With this configuration, in the state illustrated in FIG. 1 (the state before the start of use), the stopper 14 is attached to the barrel body 10, and thus the front barrel 20 does not move, and the sealing condition of the reservoir (storage portion) 11 is maintained by the plug body 12 (sealing ball 13) that seals the reservoir (storage portion) 11. As illustrated in FIG. 2, when the stopper 14 is removed at the start of use, the front barrel 20 moves in the axial direction by the length of the stopper 14 and abuts against the barrel body 10. That is, the pipe joint 22 of the front barrel 20 and the plug body 12 are joined to each other, and the sealing ball 13 attached to the plug body 12 is removed by the tip of the pipe joint 12.

As a result, the sealing state of the reservoir (storage portion) 11 is released, and the application liquid can be supplied from the reservoir 11 to the brush (application portion) 23.

An application liquid discharging mechanism 30 is provided at the rear end portion of the barrel body 10. The application liquid discharging mechanism 30 is configured to allow a piston 32 to move forward by a predetermined distance by rotating a canopy 31 and to discharge a predetermined amount of the application liquid. The brush 23 is covered with a cap 40.

In FIGS. 1 and 2, reference numeral 15 denotes a stirring ball configured to stir the application liquid. The application liquid discharging mechanism 30 is disclosed in JP 2012-157611 A, which has been proposed by the present applicant. The application liquid discharging mechanism 30 is not limited to a rotary type in which the application liquid is discharged by rotating the canopy 31, but may be a knock type in which the application liquid is discharged by pressing the canopy 31.

In the liquid cosmetic applicator A of the present disclosure configured as described above, firstly, the stopper 14 is removed, and the cap 40 (front barrel body 21) is pushed (moved) to the rear side of the barrel body 10. As a result, the end portion of the pipe joint 22 abuts against the sealing ball 13, the sealing ball 13 is removed from the plug body 52, and the application liquid can be supplied. Thereafter, the cap 40 is removed, the canopy 31 is rotated, and the piston 32 is allowed to move forward by a predetermined distance. Thus, a predetermined amount of the application liquid is discharged to the brush 23, and the application liquid is applied to an application site of the user.

The shape, structure, and material of the brush 23 to be used are not particularly limited as long as the brush 23 can be suitably applied to an eyeliner, an eyeshadow, an eyebrow, or the skin. In the embodiment of the present disclosure, the brush is formed of an aggregate of fibers (brush hairs) made of linear synthetic fibers with tapered tips and having a non-circular deformed cross-section, and is composed of a plurality of types of brush hairs having different cross-sectional shapes. The brush hairs having a deformed cross-section refer to brush hairs having a shape other than a circular shape in a plane perpendicular to the axial direction of the brush hairs. Examples of the brush hairs include those having a cross-sectional shape such as a substantially star shape, a substantially cocoon shape, a substantially cross shape, a substantially triangular shape, a substantially Y-shape, a substantially semicircular shape, or a substantially rectangular shape, and those having a shape provided with at least one or more projections. Examples of the synthetic fibers include synthetic resin fibers produced by melt-spinning of a thermoplastic polymer, for example, a polyester such as polyethylene terephthalate, polybutylene terephthalate, or a polyethylene terephthalate-isophthalate co-condensation polymer; a polyolefin such as polyethylene or polypropylene; or a polyamide such as nylon 6, nylon 610, or nylon 612.

In the liquid cosmetic applicator A of the present disclosure configured as described above, when multiple types of brush hairs having different deformed cross sections are used in the brush serving as the application portion, a decrease in number of voids (gaps) between the brush hairs is suppressed, and strong elasticity can be imparted to the brush. In addition, the above aqueous liquid cosmetic of the present disclosure does not blur on the skin even when it is applied on the skin, and contains a pigment containing carbon as a main component, which causes less dripping (dropping) and has a low viscosity. Therefore, the properties of drawing darkly can be achieved. Then, the aqueous liquid cosmetic serving as the application liquid can be sufficiently contained in the brush due to the synergistic effect of the characteristics of the aqueous liquid cosmetic composition and the structural characteristics of the liquid cosmetic composition applicator, and the aqueous liquid cosmetic can be applied comfortably. Moreover, in the present disclosure, the aqueous liquid cosmetic contains, as a colorant, a pigment such as carbon black dispersed by resin components, and thus can be claimed as a composition containing no surfactant (free of a surfactant) . Therefore, both fixation and cleansing properties of drawn lines can be highly achieved, and the resultant aqueous liquid cosmetic is suitable for a liquid cosmetic applicator of a makeup cosmetic such as an eyeshadow, an eyeliner, an eyebrow cosmetic, or a mascara.

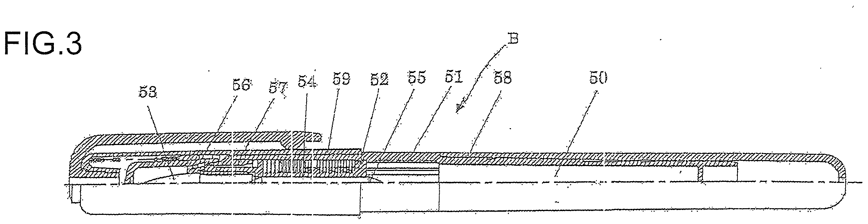

FIG. 3 illustrates a direct-liquid-type liquid cosmetic applicator of a collector type, which stores the liquid cosmetic composition of the present disclosure.

As illustrated in FIG. 3, the liquid cosmetic applicator B is, for example, an applicator including a tank portion 51 serving as a barrel that directly stores the liquid cosmetic composition 50 of the present disclosure without absorption of the liquid cosmetic composition 50 in an inner cotton or a like material. The front portion of the tank portion 51 includes a sheet body (ink holding body, collector member) 52 configured to temporarily hold the liquid cosmetic 50 pushed from the tank portion 51 when air in the tank portion 51 expands due to temperature rise, in order to prevent dropping of the liquid cosmetic 50 from a pen tip or an air pore, and the tip of the collector member 52 is provided with a brush-type pen tip (brush) 53 serving as an application body. The brush 53 having a deformed cross section used in the above liquid cosmetic applicator A can be used as the brush-type pen tip (brush) 23.

Discharging the liquid cosmetic from the tank portion 51 to the pen tip 53 is performed by discharging the liquid cosmetic 50 from the tank portion 51 to the pen tip 53 serving as the application portion via a relay core 55 having an application liquid flow channel 54 provided in a central hole of the collector member 52.

In FIG. 3, reference numerals 56 and 57 denote holder members, reference numeral 58 denotes a rear barrel fixed on the rear portion of the tank portion 51, and reference numeral 59 denotes a cap having an inner cap. For discharge of the liquid cosmetic, the rear portion of the pen tip 53 may be disposed directly in the tank portion 51 without interposing the relay core 55.

The liquid cosmetic applicator B of this embodiment can be used in the same manner as the liquid cosmetic applicator A by removing the cap 59.

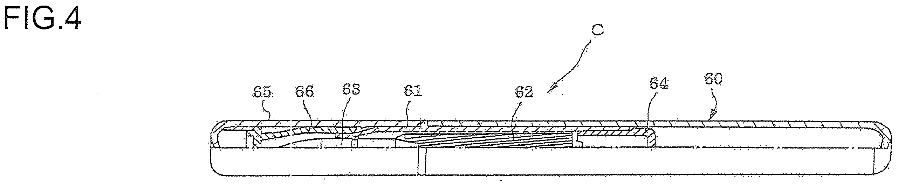

FIG. 4 illustrates a case where the aqueous liquid cosmetic of the present disclosure is used in an inner cotton-type liquid cosmetic applicator.

As illustrated in FIG. 4, the liquid cosmetic applicator C of this embodiment has, for example, a structure in which a cosmetic applicator body 60 includes an inner barrel 61; the inner barrel 61 accommodates an impregnation body 62 formed of, for example, an inner cotton impregnated with the liquid cosmetic composition of the present disclosure; the tip end of the impregnation body 62 is provided with an application body 63 formed of a brush having a deformed cross section used in the liquid cosmetic applicator A for application of the liquid cosmetic; and a tail plug 64 is fixed at the rear end of the inner barrel 61. Reference numeral 65 denotes a cap body having an inner cap portion 66. The liquid cosmetic applicator C of this embodiment can be used by removing the cap 65.

The liquid cosmetic applicator illustrated in FIG. 3 or FIG. 4 is configured as described above, and thus the liquid cosmetic applicator achieves sufficient impregnation of the brush with the aqueous liquid cosmetic of the present disclosure serving as the application liquid and enables comfortable application.

In the liquid cosmetic applicators A to C according to the embodiments of the present disclosure described above, the fiber of the brush used has a non-circular deformed cross section. As described above, the aqueous liquid cosmetic according to the present disclosure has such a low density that it does not blur on the skin even when it is applied onto the skin, causes less dripping (dropping), and enables dense drawing. Therefore, the brush used in each of the embodiments A to C may be formed of liner synthetic fibers having a tapered tip and may have a circular cross section.

The liquid cosmetic applicator of the above embodiment of the present disclosure has been described with reference to, for example, a liquid cosmetic applicator of liquid eyeliner or liquid eye shadow that is a cosmetic composition as the aqueous liquid cosmetic of the present disclosure. However, the present disclosure is not limited thereto, and the present disclosure can also be applied to an eyebrow applicator for drawing a line on eyebrows and for drawing a line on the skin. The applicator of rotary extension type illustrated in FIG. 1 is used as a liquid pressing mechanism of the liquid cosmetic applicator of the above embodiment, but a liquid cosmetic applicator of knock extension type may be used.

The aqueous liquid cosmetic of the present disclosure having such a composition is a cosmetic stored in a type of applicator in which liquid is fed from an application liquid storage portion to an application portion using capillary force. Since the aqueous liquid cosmetic contains a suitable combination of raw materials, for example, at least a pigment containing carbon as a main component, such as carbon black, a water-soluble acrylate copolymer, emulsion particles containing acrylate copolymer, and an aqueous solvent, makeup durability can be achieved even when the cosmetic contains a pigment containing carbon as a main component and does not contain a surfactant. Therefore, the thus-provided aqueous liquid cosmetic can highly achieve both fixation and cleansing properties of drawn lines, and is suitable as a makeup cosmetic such as an eyeshadow, an eyeliner, an eyebrow cosmetic, or a mascara.

### Examples

The present disclosure will next be described in further detail with reference to Examples and Comparative Examples, but the present disclosure is not limited to the following Examples.

Present Disclosure: Examples 1 to 8 and Comparative Examples 1 to 3 Cosmetic compositions as aqueous liquid cosmetics of the present disclosure having formulations shown in Table 1 below (blending unit: mass%, total amount 100 mass%) were prepared by the following method. The viscosity, pH, and surface tension of each of the liquid cosmetics were measured by the above-described measurement methods, and the liquid cosmetic was evaluated for fixation, sebum resistance, water-resistant fixation, drying property, and coating property over time by the following evaluation methods.

The results are shown in Table 1 below.

### Method for Preparing Aqueous Liquid Cosmetic

A cosmetic composition for an aqueous liquid cosmetic having the formulation shown below in Table 1 was prepared by a typically used procedure. Specifically, a pigment containing, as a main component, carbon that is a colorant and a water-soluble acrylate copolymer were added to purified water as a vehicle and were dispersed using LABSTAR (available from Ashizawa Finetech Ltd.) as a dispersing machine. Then, other components were added to the dispersion, followed by mixing to prepare a cosmetic composition.

### Method of Measuring pH

The pH of each of the prepared aqueous liquid cosmetics was measured at 25°C with a glass electrode pH meter.

### Method of Measuring Viscosity

The viscosity of each of the aqueous liquid cosmetics prepared by the above-described method was measured using an ELD-type viscometer available from Toki Sangyo Co., Ltd under with a standard rotor: 50 rpm (shear rate: 192 [s⁻¹]) at 25°C.

### Method of Evaluating Fixation

The prepared aqueous liquid cosmetic was charged in the eyeliner of FIG. 1 and was applied to the skin. Then, the applied surface was rubbed with the ball of a finger to evaluate fixation according to the following evaluation criteria.

### Evaluation criteria:

A: No change is observed in the drawn line by rubbing with the ball of a finger.
B: A slight change is observed in the drawn line by rubbing with the ball of a finger.
C: A change is observed in the drawn line by rubbing with the ball of a finger.
D: The drawn line is erased by rubbing with the ball of a finger.

### Method of Evaluating Sebum Resistance

The prepared aqueous liquid cosmetic was charged in the direct-liquid-type eyeliner of FIG. 1 and was applied to the skin. Then, artificial sebum was applied and the applied surface was rubbed with the ball of a finger to evaluate sebum resistance according to the following evaluation criteria.

### Evaluation criteria:

A: No change is observed in the drawn line by rubbing with the ball of a finger.
B: Slight change is observed in the drawn line by rubbing with the ball of a finger.
C: A change is observed in the drawn line by rubbing with the ball of a finger.
D: The drawn line is erased by rubbing with the ball of a finger.

### Method of Evaluating Water-Resistant Fixation

The prepared aqueous liquid cosmetic was charged in the direct-liquid-type eyeliner of FIG. 1 and was applied to the skin, followed by rinsing with running water. Then, the applied surface was rubbed with the ball of a finger to evaluate water-resistant fixation according to the following evaluation criteria.

### Evaluation criteria:

A: No change is observed in the drawn line by rubbing with the ball of a finger.
B: A slight change is observed in the drawn line by rubbing with the ball of a finger.
C: A change is observed in the drawn line by rubbing with the ball of a finger.
D: The drawn line is erased by rubbing with the ball of a finger.

### Method of Evaluating Drying Property

The prepared aqueous liquid cosmetic was charged in the direct-liquid-type eyeliner of FIG. 1 and was applied to the skin. After the elapse of a certain period of time, a facial tissue was pressed to the drawn line, to evaluate drying property according to the following evaluation criteria.

### Evaluation criteria:

A: No liquid adheres to the facial tissue.
B: Liquid slightly adheres to the facial tissue.
C: Liquid adheres to the facial tissue.
D: Most liquid transfers to the facial tissue.

### Method of Evaluating Liquid Retaining Property

The prepared aqueous liquid cosmetic was charged in the direct-liquid-type eyeliner of FIG. 1, and the eyeliner was placed at 50°C for one week with the brush tip facing downward. Then, the liquid retaining property of the collector type was evaluated according to the following evaluation criteria.

### Evaluation criteria:

A: No liquid leaks from the brush tip.
B: Liquid slightly leaks from the brush tip.
C: Liquid leaks from the brush tip.

**[Table 1]**

| | | | | | | | | | | (Total amount 100 mass%) | | |
|---|---|---|---|---|---|---|---|---|---|---|---|---|
| | | Example | | | | | | | | Comparative Example | | |
| | | 1 | 2 | 3 | 4 | 5 | 8 | 7 | 8 | 1 | 2 | 3 |
| Pigment | Carbon black *1 | 6.67 | 6.67 | 5.33 | 6.67 | 6.67 | 8.00 | 2.00 | 2.00 | 8.00 | 5.33 | 8.00 |
| | Aluminum powder *2 | | | | | | | 1.50 | | | | |
| | Metal oxide-coated glass powder *3 | | | | | | | | 1.50 | | | |
| Emulsion resin (particles) | Ethylhexyl acrylate/methyl methacrylate) copolymer, (acrylates/methylstyrene/styrene) copolymer ammonium *4 | 11.75 | 10.58 | 11.75 | 11.75 | 12.93 | 11.75 | 11.75 | 11.75 | | | 10.58 |
| | Acrylates copolymer *5 | | | | | | | | | 9.00 | 12.60 | |
| Water-soluble resin (pigment dispersant) | (Styrene/acrylates) copolymer *6 | 2.00 | 2.00 | 1.20 | 2.00 | 2.00 | 2.40 | 0.60 | 0.60 | | | |
| | (Acrylates/octylacrylamide) copolymer *7 | | | | | | | | | 1.20 | 0.80 | 1.20 |
| Preservative | Phenoxyethanol | 0.55 | 0.55 | 0.35 | 0.55 | 0.55 | 0.55 | 0.55 | 0.55 | 0.50 | 0.55 | 0.55 |
| | Methylparaben | 0.20 | 0.20 | 0.20 | 0.20 | 0.20 | 0.20 | 0.20 | 0.20 | 0.20 | 0.20 | 0.20 |
| | Ethylparaben | 0.13 | 0.12 | 0.13 | 0.13 | 0.13 | 0.13 | 0.13 | 0.13 | 0.10 | 0.10 | 0.10 |
| Component added | 1,2-Hexanediol | 1.50 | 1.50 | 1.50 | 1.50 | 1.50 | 1.50 | 1.50 | 1.50 | | 1.50 | 1.50 |
| | Ethylhexylglycerin | 0.12 | 0.12 | 0.12 | 0.12 | 0.12 | 0.12 | 0.12 | 0.12 | 0.12 | 0.12 | 0.16 |
| | Tocopherol | 0.10 | | | | | | | | | | |
| | Triethanolamine | | | | | | | | | 0.60 | 0.40 | 0.60 |
| | Sodium dehydroacetate | | | | | | | | | 0.33 | | |
| Aqueous solvent | 1,3-Butylene glycol | 7.65 | 7.31 | 3.87 | 9.51 | 9.50 | 9.51 | 9.50 | 9.50 | 10.00 | 10.50 | 10.50 |
| | Water (purified water) | Balance | Balance | Balance | Balance | Balance | Balance | Balance | Balance | Balance | Balance | Balance |
| Physical properties | Viscosity mPas: 50 rpm (shear rate of 192 S⁻¹) | 4 | 3.9 | 3.8 | 4.0 | 4.3 | 5.0 | 4.8 | 4.9 | 4.1 | 3.6 | 4.8 |
| | pH | 7.5 | 7.3 | 7.4 | 7.5 | 7.6 | 7.5 | 7.5 | 7.4 | 7.6 | 7.8 | 7.8 |
| | Surface tension (mN/m) | 35.4 | 35.4 | 35.4 | 35.4 | 35.4 | 35.4 | 35.5 | 35.6 | 35.8 | 34.3 | 31.0 |
| Evaluation | Fixing property | A | A | A | A | A | A | A | A | C | B | A |
| | Sebum resistance | A | A | A | A | A | A | A | A | C | C | A |
| | Water-resistant fixing property | A | A | A | A | A | A | A | A | C | B | A |
| | Drying property | B | A | B | B | A | A | A | A | C | C | B |
| | Liquid retention in collector type | A | A | A | A | A | A | A | A | A | A | C |

| | | | | | | | | | | | | |
|---|---|---|---|---|---|---|---|---|---|---|---|---|
| *1 to *7 in Table 1 above are as described below. *1: DK BLACK No. 2, available from DAITO KASEI KOGYO CO., LTD. *2: Cosmicolor Celeste Frost SL, available from Toyo Aluminum Co., Ltd. *3: Metashine MT1030RY, available from Nippon Sheet Glass Co., Ltd. *4: EMUPOLY-CE-119N, available from Gifu Shellac Mfg. Co., Ltd. *5: SYNTRAN EX149PE, available from INTERPOLYMER *6: SYNTRAN 5402 available from INTERPOLYMER *7: AMPHOMER HC, available from Nouryon | | | | | | | | | | | | |

As is clear from the results in Table 1 above, the aqueous liquid cosmetics of Examples 1 to 8, which fall within the scope of the present disclosure, are excellent in fixation, sebum resistance, water-resistant fixation, drying property, and coating property over time, as compared with the aqueous liquid cosmetics of Comparative Examples 1 to 3, which fall outside the scope of the present disclosure.

### Industrial Applicability

Provided is an aqueous liquid cosmetic that is stored in a liquid cosmetic applicator and is suitable as a makeup cosmetic such as an eyeshadow, an eyeliner, an eyebrow cosmetic, or a mascara. Reference Signs List

- 1: Applicator
- 10: Barrel body
- 11: Reservoir (storage portion)
- 12: Plug body
- 13: Sealing ball
- 14: Stopper
- 20: Front barrel
- 21: Front barrel body
- 23: Brush (application portion)

## Claims

1. An aqueous liquid cosmetic that is stored in a type of applicator in which liquid is fed from an application liquid storage portion to an application portion using a capillary force, the aqueous liquid cosmetic comprising:
at least a pigment containing carbon as a main component; a water-soluble acrylate copolymer selected from Group A below; core-shell-type emulsion particles containing acrylate copolymer; and an aqueous solvent:
Group A: (styrene/acrylate) copolymer and ammonium acrylate copolymer.

2. The aqueous liquid cosmetic according to claim 1, wherein the pigment containing carbon as a main component is selected from carbon black and/or graphite.

3. The aqueous liquid cosmetic according to claim 1 or 2, wherein the aqueous solvent is selected from water, a lower alcohol having 6 or less carbons, or a polyhydric alcohol.

4. The aqueous liquid cosmetic according to claim 3, wherein a content of the water is 50% or more.

5. The aqueous liquid cosmetic according to any one of claims 1 to 4, wherein the application portion of the applicator is a brush.
